# EUROPEAN PATENT APPLICATION

(11) **EP 3 965 113 A1**
(43) Date of publication of application: **09.03.2022**
(21) Application number: 20194556.5
(22) Date of filing: 04.09.2020
(51) Int. Cl.: G16H 20/30, G16H 50/20, G16H 50/30

(54) **METHOD FOR EVALUATING THE EFFECTIVENESS OF CPR IN A SMARTPHONE OR SMARTWATCH**

(71) Applicant: Lightbringer Digital-Health GmbH, 22081 Hamburg (DE)
(72) Inventor: Leuchte, Alexander, 22081 Hamburg (DE); Klimczak, Mateusz, 80-297 Banino (PL)
(74) Representative: Schmitt-Nilson Schraud Waibel Wohlfrom Patentanwälte Partnerschaft mbB

(57) **Abstract**

A method for evaluating the effectiveness of CPR in a smartphone (2) or smartwatch (402) includes: obtaining a sequence (10) of acceleration values (14) from acceleration data gathered by an acceleration sensor (306) of the smartphone (2) or smartwatch (402); relating the sequence (10) of acceleration values (14) to a pattern of threshold values (60, 62, 64, 70, 72, 74); and on the basis of said relating of the sequence (10) of acceleration values (14) to the pattern of threshold values (60, 62, 64, 70, 72, 74), determining an indication whether an effective compression depth has been reached.

## Description

The present invention is in the field of tools that help users to perform cardiopulmonary resuscitation (CPR), herein also referred as cardiac massage. In particular, the present invention relates to methods and devices for evaluating the effectiveness of CPR.

Cardiac arrest is a very severe medical condition and requires immediate attention. According to statics for Europe and North America, hundreds of thousands of cardiac arrests occur annually outside the hospital environment. In most of these cases, no medically trained personal is around, leaving random first responders with the task of performing CPR. Such non-trained persons are commonly hesitant to perform CPR or even refuse to take action. Reasons for failing to help are fear of doing harm, no training in CPR, lack of confidence, and fear of being sued. Further, when taking action, un-trained persons are prone to perform CPR in a non-effective manner.

Accordingly, it would be beneficial to provide tools for helping persons in performing effective CPR. In particular, it would be helpful to provide tools that provide intuitive help to the person performing CPR.

Exemplary embodiments of the invention include a method for evaluating the effectiveness of CPR in a smartphone, the method comprising: obtaining a sequence of acceleration values from acceleration data gathered by an acceleration sensor of the smartphone; relating the sequence of acceleration values to a pattern of threshold values; and on the basis of said relating of the sequence of acceleration values to the pattern of threshold values, determining an indication whether an effective compression depth has been reached.

Exemplary embodiments of the invention further include a method for evaluating the effectiveness of CPR in a smartwatch, the method comprising: obtaining a sequence of acceleration values from acceleration data gathered by an acceleration sensor of the smartwatch; relating the sequence of acceleration values to a pattern of threshold values; and on the basis of said relating of the sequence of acceleration values to the pattern of threshold values, determining an indication whether an effective compression depth has been reached.

Exemplary embodiments of the invention allow for a person performing CPR to use their smartphone to control the effectiveness in applying CPR. With the method for evaluating the effectiveness of CPR, acceleration data, which is available in modern smartphones due to the inclusion of acceleration sensors therein, may be used for evaluating the effectiveness of CPR. The effectiveness of CPR is derived from the acceleration data that is gathered by the acceleration sensor of the smartphone. By placing the smartphone between the chest of the person in cardiac arrest, herein also referred to as patient, and the hands of the person performing CPR, herein also referred to as caregiver, the smartphone is subject to the acceleration exerted by the person performing the CPR and the method for evaluating the effectiveness of CPR may determine a reliable indication regarding the effectiveness thereof. With smartphones becoming more and more ubiquitous in our modern world and with the likelihood of a random first responder having a smartphone continuously increasing, the method for evaluating the effectiveness of CPR in accordance with exemplary embodiments of the invention may enable a rapid creation of an infrastructure of effective tools for helping first responders in performing CPR. Similar considerations apply to the use of a smartwatch to control the effectiveness in applying CPR. Modern smartwatches also include acceleration sensors. When performing CPR, the smartwatch may be removed from the caregiver's wrist and placed on the patient's chest, similar to the smartphone. It is also possible that the the smartwatch remains on the caregiver's wrist, in which case the wrist of the caregiver and, thus, the smartwatch may be subject to a highly similar acceleration as the hands of the caregiver. The acceleration, as sensed by the smartwatch, may be a good proxy for the acceleration exerted by the caregiver to the patient's chest, and the method for evaluating the effectiveness of CPR may also allow for a reliable indication regarding the effectiveness of CPR.

Exemplary embodiments of the invention allow for evaluating the effectiveness of CPR in a smartphone / smartwatch, i.e. via the help of / by means of a smartphone / smartwatch. The term CPR, i.e. cardiopulmonary resuscitation, refers to the application of a sequence of compression cycles to a patient's chest. The method for evaluating the effectiveness of CPR may determine the indication whether an effective compression depth has been reached on the basis of a single CPR compression cycle or a portion of a single CPR compression cycle or on the basis of a plurality of CPR compression cycles or portions of a plurality of CPR compression cycles. A CPR compression cycle is defined as the combination of a pressure phase and a release phase, i.e. the combination of a downward motion and an upward motion, when assuming that the patient is lying on the ground with his/her back on the ground. The method for evaluating the effectiveness of CPR may be carried out in a repetitive manner. For example, a new indication whether an effective compression depth has been reached may be determined in every CPR compression cycle.

Exemplary embodiments of the invention allow for evaluating the effectiveness of CPR in a smartphone. The term smartphone includes any mobile device that combines cellular communication and mobile computing functions and that is intended to be carried around and used on a daily basis. The smartphone is a mobile device that allows for voice calls, data-based internet services, and potentially various other types of services. The smartphone may have a substantially rectangular shape. The longer side of the substantially rectangular shape may for example have between 10 cm and 20 cm and/or the shorter side of the substantially rectangular shape may for example have between 5 cm and 10 cm. With such extensions, the smartphone may be well-suited for being placed between the patient's chest and the hands of the person performing CPR and may have a sufficient size for housing the computing, memory, and sensor components for carrying out the method for evaluating the effectiveness of CPR.

Exemplary embodiments of the invention allow for evaluating the effectiveness of CPR in a smartwatch. The term smartwatch includes any mobile device that combines wireless communication functions and mobile computing functions and that is intended to be carried on a user's wrist during normal daily routines. The smartwatch may also have cellular communication functions. The smartwatch may allow for voice calls, data-based internet services, and potentially various other types of services.

The method for evaluating the effectiveness of CPR in a smartphone / smartwatch comprises obtaining a sequence of acceleration values from acceleration data gathered by an acceleration sensor of the smartphone / smartwatch. As stated above, most modern smartphones / smartwatches have a built-in acceleration sensor. Thus, the acceleration sensor is in the smartphone / smartphone and is part of the smartphone / smartwatch. Obtaining the sequence of acceleration values may include any way of obtaining a time series of acceleration values from the raw acceleration data provided by the acceleration sensor. For example, obtaining the sequence of acceleration values may include querying the acceleration sensor and receiving acceleration data in response to said querying. It may also include reading in acceleration values from a continuous stream of acceleration data, provided from the acceleration sensor. The obtaining of the sequence of acceleration values may also include further processing steps, such as thinning out / sub-sampling the acceleration data received from the acceleration sensor. It may also include normalizing the acceleration data, such as removing the acceleration due to gravity from the acceleration data. The sequence of acceleration values are a series of acceleration values that are discretized in time, e.g. that have a defined time increment between each other.

The method for evaluating the CPR in a smartphone / smartwatch comprises relating the sequence of acceleration values to a pattern of threshold values. The expression of relating the sequence of acceleration values to a pattern of threshold values encompasses any of relating individual acceleration values to threshold values, relating aggregate values, derived from the sequence acceleration values, to threshold values, relating selected ones of the acceleration values to threshold values, and relating portions of the sequence of acceleration values, either individually or in an aggregate manner, to threshold values. The expression of a pattern of threshold values relates to a plurality of threshold values that are applied to different portions of the sequence of acceleration values over a CPR compression cycle. The pattern of threshold values may be a plurality of threshold values that the acceleration values of a CPR compression cycle, either individually or in an aggregate manner, are compared to. The pattern of threshold values can be thought of as a time series of threshold values, with each of the time series of threshold values being applied to individual ones or aggregate values of the acceleration values during a particular CPR compression cycle.

The method for evaluating the effectiveness of CPR in a smartphone / smartwatch comprises determining an indication whether an effective compression depth has been reached, with said determination being based on the relating of the sequence of acceleration values to the pattern of threshold values. The indication whether an effective compression depth has been reached does not have to be an accurate result for the compression depth. The indication may be a guess / an estimate whether an effective compression depth has been reached, with the guess / estimate being a sophisticated guess / estimate on the basis of the relating of the sequence of acceleration values to the pattern of threshold values. The relating of the sequence of acceleration values to the pattern of threshold values may yield an estimate of the compression depth, such as an estimate of the depth, for example in centimeters, that the patient's chest has been compressed. In other words, it is possible that, as a result of relating the sequence of acceleration values to the pattern of threshold values, an estimate for the compression depth is determined. This estimate may be related to a desired compression depth, in order to determine the indication whether an effective compression depth has been reached. According to medical guidelines for CPR, an ideal compression depth is between 5 cm and 6 cm. It may therefore be an option to determine whether the estimate for the compression depth is around said desired compression depth, for example within a desired compression depth interval, which may be between 4 cm and 7 cm. It is also possible not to estimate a unit length value for the compression depth, but to determine the indication whether an effective compression depth has been reached from other criteria. For example, one or more conditions may be defined regarding the relation between the sequence of acceleration values and the pattern of threshold values, in order for the indication whether an effective compression depth has been reached to be positive or negative. Exemplary ways of determining the indication whether an effective compression depth has been reached, without calculating an estimate of the actual compression depth, will be laid out hereinbelow.

According to a further embodiment, the pattern of threshold values comprises upper threshold values and lower threshold values. The step of relating the sequence of acceleration values to the pattern of threshold values may include relating the sequence of acceleration values, individually and/or in the form of aggregate values, to both upper threshold values and lower threshold values. The upper threshold values and lower threshold values may define a desired value corridor. In case the acceleration values and/or subsets of the acceleration values and/or certain portions of the acceleration values and/or aggregate values, derived from the acceleration values, fall within such desired value corridor, a positive indication that an effective compression depth has been reached may be determined. In this way, the pattern of threshold values may allow for checking against both a compression depth that is too deep and a compression depth that is too shallow.

According to a further embodiment, said relating of the sequence of acceleration values to the pattern of threshold values comprises: grouping a subset of the sequence of acceleration values into a plurality of acceleration value blocks; for each of the plurality of acceleration value blocks, determining a block-specific aggregate value; and for each of the block-specific aggregate values, comparing the block-specific aggregate value with at least one block-specific threshold value. In this way, a good compromise between a high accuracy in determining the indication whether an effective compression depth has been reached and a good handling of outlier acceleration values, which may be the result of the complex interaction between the pressure exerted by a person performing CPR and a human chest, may be achieved. The indication whether an effective compression depth has been reached may be determined in a highly reliable manner. For each of the plurality of acceleration value blocks, there is at least one block-specific threshold value. In a particular embodiment, there may be an upper block-specific threshold value and a lower block-specific threshold value for each of the plurality of acceleration value blocks. The block-specific threshold values may be different for the different blocks of acceleration values. The set of block-specific threshold values are an example of a pattern of threshold values, to which the sequence of acceleration values are related. The plurality of acceleration value blocks may have fixed block lengths, such as 10 acceleration values per acceleration value block or any other suitable number of acceleration values per acceleration value block, and the block-specific threshold values may be set values. It is also possible that the block length may be set / adapted on the basis of the sequence of acceleration values. For example, on the basis of the highest value and/or the lowest value and/or a particular spike in a CPR compression cycle, the block length may be set and the block-specific threshold values may adapted in accordance therewith. In this way, an adaptation of the method to the particular patient may be achieved.

The subset of the sequence of acceleration values may be a set number of acceleration values. For example, the subset of acceleration values may be the number of acceleration values that correspond to a pressure phase of a CPR compression cycle or to a release phase of a CPR compression cycle or to a combined pressure and release phase of a CPR compression cycle. This number may be set assuming an ideal duration of the CPR compression cycle. The ideal duration may be assumed to be 0.6 seconds. This ideal duration of the CPR compression cycle is based on the medical guidelines that recommend 100 CPR compression cycles per minute for a particularly effective CPR. It is also possible to look at a time frame that is defined / set in a different manner. The actual number of acceleration values in the subset of the sequence of acceleration values may be determined in accordance with the sampling rate of the sequence of acceleration values.

The selection of the subset of the sequence of acceleration values may be carried out in response to a trigger. For example, the trigger may be the beginning or the half-way point or the end point of a CPR compression cycle, as deduced from the sequence of acceleration values. The subset may then contain above described set number of acceleration values after the trigger.

According to a further embodiment, said determining of the block-specific aggregate value comprises applying an aggregation function to the acceleration values in the given acceleration value block. The aggregation function may be the same for the plurality of acceleration value blocks or may be different for the acceleration value blocks at different positions in the subset of the sequence of acceleration values. The aggregation function may in particular be a deterministic operation that transforms multiple acceleration values, as given in an acceleration value block, into a single aggregate value, which may be compared to one or more block-specific threshold values. The aggregation function may comprise various mathematical operations, such as multiplication / division of acceleration values with predetermined factors, normalization of acceleration values, adding / subtracting predetermined values to / from the acceleration values, summing acceleration values, integrating acceleration values, etc. The block-specific threshold values may be adapted to the particular aggregation function used.

According to a further embodiment, said applying of the aggregation function comprises forming a sum of the acceleration values in the given acceleration value block. The sum may be a weighted sum or an un-weighted sum of the acceleration values in the given acceleration value block. Also, the sum may be a sum of the acceleration values, as they are, or a sum of the acceleration values, multiplied with / divided by a predetermined factor and/or having a predetermined value added thereto / subtracted therefrom. By forming a sum of the acceleration values in the acceleration value block in question, a smoothing effect may be achieved, with individual outliers in the sequence of acceleration values being taken into account, but being prevented from dominating the determination of the indication whether an active compression depth has been reached.

According to a further embodiment, said comparing of the block-specific aggregate value with at least one block-specific threshold value comprises determining whether the block-specific aggregate value is between a respective upper block-specific threshold value and a respective lower block-specific threshold value. In this way, a desired aggregate value corridor may be established, with respective upper and lower block-specific threshold values being given on a block-by-block basis. Above described compromise between high accuracy and manageable sensitivity to the complexities of the CPR framework may be combined with detecting both too deep and too shallow compression depths. The term respective in the expressions respective upper block-specific threshold value and respective lower block-specific threshold value refers to the position of the acceleration value block in question in the sequence of the plurality of acceleration value blocks.

According to a further embodiment, said determining of the indication whether an effective compression depth has been reached comprises determining whether the block-specific aggregate values of all acceleration value blocks of a pressure phase of a CPR compression cycle are between the respective upper block-specific threshold values and the respective lower block-specific threshold values. In other words, if the block-specific aggregate values of all acceleration value blocks are in the desired value corridor for a pressure phase of a CPR compression cycle, a positive indication that an effective compression depth has been reached is determined. It is understood that a new indication, potentially indicating that no effective compression depth has been reached, may be determined in the pressure phase of the next CPR compression cycle or in the pressure phase of the next but one CPR compression cycle, etc., depending on how often the effectiveness of CPR is evaluated in accordance with the particular implementation of the method.

By basing the indication on whether an effective compression depth has been reached on the pressure phase of the CPR compression cycle, the method takes that part of the CPR compression cycle into particular account that has the highest risk of being carried out in an ineffective manner. In particular, in case the person performing the CPR is hesitant to push hard enough into the patient's chest and/or in case the person performing the CPR presses to hard and is in danger of causing additional severe harm to the patient, the determined indication may catch such issues.

While it has been described that the method determines whether the block-specific aggregate values of all acceleration value blocks of a pressure phase of a CPR compression cycle are between the respective upper block-specific threshold values and the respective lower block-specific threshold values, the determining of the indication whether an effective compression depth has been reached may also allow one or two or a certain low percentage of block-specific aggregate values to be outside the desired value corridor and still provide a positive indication that an effective compression depth has been reached. Requiring the block-specific aggregate values of all acceleration value blocks to be inside the desired value corridor vs. allowing a certain number of block-specific aggregate values to be outside the desired value corridor is a way of tweaking a particular implementation of the method towards accuracy or towards effective handling of outlier acceleration values. A particular set-up may be selected for the particular implementation of the method, for example taking into account the length of the acceleration value blocks, i.e. the number of acceleration values in the acceleration value blocks. In exemplary embodiments with very short block lengths, such as block lengths of two or three acceleration values, and/or in exemplary embodiments of comparing the acceleration values to threshold values on an individual basis, more instances of acceleration values / block-specific aggregate values being outside the desired value corridor may be acceptable.

According to a further embodiment, the pattern of threshold values, in particular the block-specific threshold values, may be dependent on individual patient data. For example, the pattern of threshold values, in particular the block-specific threshold values, may be dependent on the sex and/or age and/or weight and/or other body parameters of the person receiving CPR. In this way, the indication whether an effective compression depth has been reached may be determined in a more granular and more targeted manner.

According to a further embodiment, a sampling rate of the sequence of acceleration values is at least 50 Hz, in particular between 50 Hz and 500 Hz, further in particular between 100 Hz and 200 Hz. The term sampling rate relates to the number of acceleration values in the sequence of acceleration values that correspond to one second of CPR. While a fairly reliable indication whether an effective compression depth has been reached may be achieved with a sampling rate of 50 Hz, it has been found that a sampling rate of 100 Hz or more provides for a much higher degree of reliability of the indication whether an effective compression depth has been reached. A higher sampling rate inherently provides more information about the CPR compression cycles applied to the chest of the patient. Also, higher sampling rates provide for a higher degree of freedom in grouping acceleration values into acceleration value blocks. It has been found that a particularly good compromise between reliability and computational complexity may be achieved at sampling rates between 100 Hz and 200 Hz.

According to a further embodiment, said obtaining of the sequence of acceleration values from acceleration data gathered by the acceleration sensor of the smartphone comprises obtaining a sequence of acceleration values in the z-direction of the smartphone. The z-direction of the smartphone is understood as the direction perpendicular to the main plane of extension of the smartphone, e.g. the direction perpendicular to the main display of the smartphone. Assuming that the smartphone is positioned flat on the patient's chest during the performance of CPR, the z-direction of the smartphone corresponds to the true compression direction into the patient's chest during the performance of CPR. The obtaining of the sequence of acceleration values in the z-direction of the smartphone may thus provide highly accurate acceleration values towards the patient's chest in the CPR compression phase and away from the patient's chest during the CPR release phase. The above described data processing steps and operations, described in the context of the sequence of acceleration values, may be carried out on the basis of the sequence of acceleration values in the z-direction of the smartphone. While the concentration on the acceleration values in the z-direction of the smartphone may be dispensed with and a fairly reliable indication whether an effective compression depth has been reached may be achieved on the basis of the total acceleration values, inter alia because the person receiving CPR is supposed to lie flat on their back on an even ground, obtaining a sequence of acceleration values in the z-direction of the smartphone may greatly increase the reliability of the indication whether an effective compression depth has been reached. This is particularly true in situations where the patient is positioned on an uneven ground / supporting surface and/or where the patient is positioned on a hill or other inclined ground structure. Analogous considerations apply to the method for evaluating the effectiveness of CPR in a smartwatch, when the smartwatch is positioned flat on the patient's chest during the performance of CPR.

According to a further embodiment, obtaining the sequence of acceleration values in the z-direction of the smartphone comprises using gyroscope data, gathered by a gyroscope of the smartphone, for determining a tilt of the smartphone. With the gyroscope of the smartphone, a tilt of the smartphone, in particular a tilt of the main plane of extension of the smartphone with respect to the horizontal plane, may be determined. On the basis of said determination of the tilt of the smartphone with respect to the horizontal plane in the earth frame of reference, the z-component of the raw acceleration data, gathered by the acceleration sensor of the smartphone, can be reliably and efficiently determined. The raw acceleration data, gathered by the acceleration sensor of the smartphone, may be provided in the earth frame of reference. The gyroscope of the smartphone may be any kind of gyroscope-type sensor. The term gyroscope may refer to any type of sensor that allows for determining the orientation of the smartphone with respect to the earth frame of reference, i.e. with respect to an earth coordinate system. In case the raw acceleration data, gathered by the acceleration sensor of the smartphone, is already provided in the smartphone frame of reference, the obtaining of the sequence of values in the z-direction of the smartphone may comprise using the z-component of the acceleration data and discarding the x-component and y-component of the acceleration data, gathered by the acceleration sensor of the smartphone. Analogous considerations apply to the method for evaluating the effectiveness of CPR in a smartwatch, when the smartwatch is positioned flat on the patient's chest during the performance of CPR.

According to a further embodiment, said obtaining of the sequence of acceleration values from acceleration data gathered by the acceleration sensor of the smartwatch comprises obtaining a sequence of acceleration values in an estimated compression direction. The estimated compression direction may be the upwards / downwards direction in the earth frame of reference. It is also possible that the estimated compression direction is the direction of the largest acceleration values in the sequence of acceleration values. In this way, the unknown orientation of the smartwatch, when worn on the caregiver's wrist during the performance of CPR, may be accounted for, and the acceleration values in the estimated compression direction may be used for the further signal processing. In particular, the above described data processing steps and operations, described in the context of the sequence of acceleration values, may be carried out on the basis of the sequence of acceleration values in the estimated compression direction. Using the upwards / downwards direction in the earth frame of reference as the estimated compression direction is based on the assumption that the patient is lying flat on their back on an even ground, which is the recommended CPR position. Using the direction of the largest acceleration values as the estimated compression direction is based on the assumption that the largest motion by the caregiver's hands is attributable to compressing the patient's chest, when performing CPR.

According to a further embodiment, said obtaining a sequence of acceleration values in an estimated compression direction comprises using gyroscope data, gathered by a gyroscope of the smartwatch, for determining a tilt of the smartwatch. With the gyroscope of the smartwatch, a tilt of the smartwatch, in particular a tilt of the main plane of extension of the smartwatch with respect to the horizontal plane in the earth frame of reference, may be determined.

According to a further embodiment, the method further comprises: examining the sequence of acceleration values for a predetermined acceleration spike pattern; and, on the basis of said examining of the sequence of acceleration values for the predetermined acceleration spike pattern, assessing a reliability of the indication whether an effective compression depth has been reached. The expression of examining the sequence of acceleration values for a predetermined acceleration spike pattern relates to analyzing the sequence of acceleration values with respect to the predetermined acceleration spike pattern. This step also be referred to as performing signal processing on the sequence of acceleration values in the form of relating the sequence of acceleration values to a predetermined acceleration spike pattern. The finding of the predetermined acceleration spike pattern in the sequence of acceleration values may result in a positive assessment of an indication that an effective compression depth has been reached. In other words, in case above described method steps provide for a positive indication that an effective compression depth has been reached, the finding of the predetermined acceleration spike pattern may provide for a secondary validation / verification that an effective compression depth has been reached. Conversely, in case above described method steps provide for a positive indication that an effective compression depth has been reached, but no predetermined acceleration spike pattern can be found in the sequence of acceleration values, the initial indication that an effective compression depth has been reached may be assessed to be not reliable. Such a finding may result in updating the indication whether an effective compression depth has been reached. The updated indication may be an indication that no effective compression depth has been reached or may be an indefinite indication as to whether an effective compression depth has been reached or may be of any other form that reflects the negative validation / verification via the predetermined acceleration spike pattern.

With the predetermined acceleration spike pattern, the sequence of acceleration values may be compared with an expected behavior of the human chest during CPR. For example, the predetermined acceleration spike pattern may represent a typical resistance behavior of the ribs of the human chest against compressive forces. In this way, the predetermined acceleration spike pattern may indicate that the sequence of acceleration values is comparable to what is expected from a human chest. The examination of the sequence of acceleration values for the predetermined spike pattern may allow for a distinction between an actual effective compression of the human chest vs. a pushing of the human chest into a soft ground, such as into a mattress, or a pushing of the human body down a hill or other inclined structure or other scenarios where the human chest is moved a desirable distance, but no effective compression of the chest takes place.

According to a further embodiment, the predetermined acceleration spike pattern comprises an initial acceleration spike in a pressure phase of a CPR compression cycle and a follow-up acceleration spike in the same pressure phase of the same CPR compression cycle. The initial acceleration spike and the follow-up acceleration spike are understood as acceleration spikes in the same direction, i.e. in the direction towards the patient's chest in the pressure phase, which is also referred to as the negative z-direction in the smartphone frame of reference, assuming that the smartphone's front side is facing upwards, or referred to as estimated compression direction as determined by the smartwatch. The initial acceleration spike may be defined as the first instance that the acceleration in the negative z-direction is larger than a predefined first acceleration spike threshold. The follow-up acceleration spike may be the first instance that the acceleration in the negative z-direction is larger than a predefined second acceleration spike threshold, after the acceleration has returned at least to an inter-spike threshold value. The predetermined acceleration spike pattern may be a w-type acceleration spike pattern, with the second low point of the w-type pattern being smaller in magnitude than the first low point of the w-type pattern. The presence of such a w-type pattern may be a good indication that the patient's chest is pressed through a characteristic behavior of the human ribs. The predetermined acceleration spike pattern may also have another suitable form that allows for assessing the reliability of the indication whether an effective compression depth has been reached. For example, the predetermined acceleration spike pattern may be a v-type acceleration spike pattern or an m-type acceleration spike pattern.

While it has been described that the examining of the sequence of acceleration values for a predetermined acceleration spike pattern is used for providing an additional validation / verification that an effective compression depth has been reached, the examining of the sequence of acceleration values may also form the original / sole basis for determining an indication whether an effective compression depth has been reached. In other words, the indication whether an effective compression depth has been reached may be determined on the basis of the examining of the sequence of acceleration values for a predetermined acceleration spike pattern, without relating the sequence of acceleration values to the pattern of threshold values. Exemplary embodiments of the invention thus further include a method for evaluating the effectiveness of CPR in a smartphone / smartwatch, the method comprising: obtaining a sequence of acceleration values from acceleration data gathered by an acceleration sensor of the smartphone / smartwatch; examining the sequence of acceleration values for a predetermined acceleration spike pattern; and, on the basis of said examining of the sequence of acceleration values for the predetermined acceleration spike pattern, determining an indication whether an effective compression depth has been reached. The additional features, modification, and effects, as described herein with respect to the method for evaluating the effectiveness of CPR in a smartphone / smartwatch, also relate to these embodiments of the invention.

According to a further embodiment, the method further comprises determining at least one of a CPR compression cycle duration and a CPR compression cycle frequency from the sequence of acceleration values. The CPR compression cycle duration / CPR compression cycle frequency may be determined as a further indication whether the performed CPR is effective. While not being directly related to whether an effective compression depth has been reached, the CPR compression cycle duration / CPR compression cycle frequency provides for another dimension in evaluating the effectiveness of CPR in the smartphone / smartwatch. The determined CPR compression cycle duration / CPR compression cycle frequency may be used for guiding the person in performing CPR. It may also be used as an input to above discussed method steps for reaching the indication whether an effective compression depth has been reached. For example, a suitable CPR compression cycle duration / CPR compression cycle frequency may be considered a prerequisite for carrying out the determining of the indication whether an effective compression step has been reached. In this way, the method may aim at first guiding the person performing CPR to adhere to a suitable compression rhythm, before evaluating the compression depth. It is also possible to use the determined CPR compression cycle duration / CPR compression cycle frequency to perform signal processing on the sequence of acceleration values, before above discussed method steps for determining the indication whether an effective compression step has been reached are carried out. For example, the sequence of acceleration values may be normalized along the time axis in accordance with the CPR compression cycle duration / CPR compression cycle frequency. In this way, the determining of the indication whether an effective compression depth has been reached may give some leeway to sequences of acceleration values over time that would not lead to an effective compression depth under the assumption of an ideal CPR compression cycle duration / frequency. Taking into account the particular CPR compression cycle duration / frequency used, the compression depth may still be deemed effective.

According to a further embodiment, the method further comprises providing at least one of an acoustic output, a visual output, and a vibratory output via the smartphone or smartwatch, the at least one of the acoustic output, the visual output, and the vibratory output being based on at least one of the indication whether an effective compression depth has been reached, the CPR compression cycle duration, and the CPR compression cycle frequency. Each one or any subset or all of an acoustic output, a visual output, and a vibratory output may provide an effective way for providing feedback to a person performing CPR and, thus, for guiding this person through CPR and improving the effectiveness of their CPR. The acoustic / visual / vibratory output may reflect the indication whether an effective compression depth has been reached and/or may convey additional information why the compression depth is not considered effective / how the compression depth should be adjusted. The acoustic / visual / vibratory output may also contain an indication whether the determined CPR compression cycle duration / frequency is in a desired range and/or additional information indicating why the CPR compression cycle duration/CPR compression cycle frequency is not in the desired range.

The acoustic output may comprise human language outputs that guide the person performing CPR through the CPR and aim at bringing the compression depth and/or the CPR compression cycle duration / frequency to effective values. The human language outputs may be in the form of spoken smartphone / smartwatch outputs, such as "press harder", "press deeper", "stronger", "too strong", "press faster", "press slower", "faster", "slower", etc. The visual output may be a highly intuitive, uniform illumination of the screen, such as a full illumination in green or a full illumination in red. With the person performing CPR having their hands on the smartphone screen and thus covering a large portion thereof, the full illumination of the screen may increase the likelihood of this feedback being perceived by the person performing CPR. Further, with the eyes of the person performing CPR and the display of the smartwatch at that person's wrist potentially not having an ideal orientation with respect to each other, the full illumination of the screen may increase the likelihood of this feedback being perceived by the person performing CPR. It is possible that the output, in particular the acoustic output and/or the visual output, is provided as a general feedback, taking into account a past CPR compression cycle or a plurality of past CPR compression cycles. However, it is also possible that the acoustic and/or visual output provides immediate feedback regarding a current CPR compression cycle. For example, the acoustic and/or visual output may indicate a desired CPR compression cycle duration / frequency in a metronome-like manner.

The vibratory output may be used to provide immediate feedback regarding a current CPR compression cycle. For example, the smartphone / smartwatch may start to vibrate, as soon as the method estimates that the effective compression depth has been reached. While probably not having an immediate guiding effect for the present CPR compression cycle, such vibratory feedback may train a person performing CPR towards a desired compression depth.

It is pointed out that all of above discussed methods take place within the smartphone / smartwatch and do not interact with the patient in the form of a surgical, therapeutical or diagnostic method. No tissue or other matter is taken from the human body, examined, and/or manipulated. Also, no data from the human body is used for diagnostic purposes. The method solely relies on the acceleration data, generated within the smartphone / smartwatch, resulting from the acts performed by the person applying CPR, and not stemming from the patient's acts. The patient is per definition unconscious, so that the patient cannot actively interact with the smartphone / smartwatch. The steps within the smartphone / smartwatch relate to evaluating and guiding the person performing CPR, but cannot have a therapeutic effect on the patient. The therapeutic effect has to be achieved by the person performing CPR.

Exemplary embodiments of the invention further include a method of performing CPR on a patient in cardiac arrest, comprising: placing a smartphone on the chest of the patient; pressing the smartphone into the chest of the patient; evaluating the effectiveness of the CPR in the smartphone in accordance with any of the embodiments of the method for evaluating the effectiveness of CPR, as described above; and providing feedback regarding the effectiveness of the CPR via at least one of an acoustic output, a visual output, and a vibratory output from the smartphone. The additional features, modifications and effects, as discussed above with respect to the method for evaluating the effectiveness of CPR in a smartphone, apply to the method of performing CPR on a patient in cardiac arrest in an analogous manner. Said pressing of the smartphone into the chest of the patient may comprise a repeated pressing of the smartphone into the chest of the patient, in particular a repeated pressing of the smartphone into the chest of the patient between 80 times and 120 times per minute. The evaluating of the effectiveness of the CPR in the smartphone may be carried out each time the smartphone is pressed into the chest of the patient. In other words, the evaluating of the effectiveness of the CPR in the smartphone may be carried out during each CPR compression cycle. The method may also be carried out by placing a smartwatch on the chest of the patient, pressing the smartwatch into the chest of the patient, and evaluating the effectiveness of the CPR in the smartwatch in accordance with any of the embodiments of the method for evaluating the effectiveness of CPR, as described above.

Exemplary embodiments of the invention further include a method of performing CPR on a patient in cardiac arrest, comprising: pressing a caregiver's hands into the chest of the patient, with the caregiver wearing a smartwatch at his/her wrist; evaluating the effectiveness of the CPR in the smartwatch in accordance with any of the embodiments of the method for evaluating the effectiveness of CPR, as described above; and providing feedback regarding the effectiveness of the CPR via at least one of an acoustic output, a visual output, and a vibratory output from the smartwatch. The additional features, modifications and effects, as discussed above with respect to the method for evaluating the effectiveness of CPR in a smartwatch, apply to the method of performing CPR on a patient in cardiac arrest in an analogous manner. The evaluating of the effectiveness of the CPR in the smartwatch may be carried out during each CPR compression cycle.

Exemplary embodiments of the invention further include a computer program for a smartphone, the computer program containing program instructions which, when executed on a smartphone, carry out a method for evaluating the effectiveness of CPR in accordance with any of the embodiments of the method for evaluating the effectiveness of CPR in a smartphone, as discussed above. The expression of the computer program containing program instructions that carry out said method includes the computer program carrying out method steps, when being run on the processor of the smartphone, and/or the computer program, when run on the processor of the smartphone, causing method steps to be carried out by other hardware components of the smartphone. For example, the computer program may cause the speaker(s) and/or the display of the smartphone to provide the acoustic output and/or the visual output. The computer program may be a smartphone application, also referred to as smartphone app, or may be part of a smartphone application. The smartphone application may be downloadable from a smartphone application store and/or may be pre-installed on the smartphone. The computer program may also be part of a pre-installed kit of functionalities provided by the smartphone. The computer program may also be referred to as a piece of software.

Exemplary embodiments of the invention further include a computer program for a smartwatch, the computer program containing program instructions which, when executed on a smartwatch, carry out a method for evaluating the effectiveness of CPR in accordance with any of the embodiments of the method for evaluating the effectiveness of CPR in a smartwatch, as discussed above. Above considerations regarding the computer program for a smartphone apply to the computer program for a smartwatch in an analogous manner.

Exemplary embodiments of the invention further include a smartphone, configured to perform a method for evaluating the effectiveness of CPR in accordance with any of the embodiments of the method for evaluating the effectiveness of CPR in a smartphone, as described above. The additional features, modifications, and effects, as discussed above with respect to the method for evaluating the effectiveness of CPR in a smartphone, apply to the smartphone in an analogous manner. The expression of the smartphone being configured to perform said method means that the smartphone has a computer program / software installed thereon that is capable of carrying out said method. The smartphone is in particular configured to perform said method, when the smartphone is placed on a patient's chest and accelerated into and out of the patient's chest by a person performing CPR on the patient and pressing the smartphone into the patient's chest.

Exemplary embodiments of the invention further include a smartwatch, configured to perform a method for evaluating the effectiveness of CPR in accordance with any of the embodiments of the method for evaluating the effectiveness of CPR in a smartwatch, as described above. Above considerations regarding the smartphone apply to the smartwatch in an analogous manner. The smartwatch is in particular configured to perform said method, when the smartwatch is worn on a caregiver's wrist and thus experiences the same or a very similar motion as the caregiver's hands, when performing CPR on a patient.

Further exemplary embodiments of the invention are described with respect to the accompanying drawings, wherein:
Fig. 1 shows a smartphone in accordance with an exemplary embodiment of the invention in a schematic perspective view;
Fig. 2 shows the smartphone of Fig. 1, placed on a patient's chest during application of CPR by a caregiver, in a schematic view;
Fig. 3 illustrates a sequence of acceleration values, as obtained by a smartphone during performance of CPR, which form the basis for a method for evaluating the effectiveness of CPR in a smartphone in accordance with an exemplary embodiment of the invention;
Fig. 4 shows an extract of the sequence of acceleration values of Fig. 3 in an enlarged view;
Fig. 5 shows the sequence of acceleration values of Fig. 3 with highlighted acceleration spike patterns;
Fig. 6 illustrates a relating of aggregate values, derived from the acceleration values of Fig. 3, to a pattern of threshold values;
Fig. 7 shows a smartphone in accordance with an exemplary embodiment of the invention in a block diagram;
Fig. 8 shows a smartwatch in accordance with an exemplary embodiment of the invention in a schematic perspective view; and
Fig. 9 shows the smartwatch of Fig. 8, worn on a caregiver's wrist during application of CPR by a caregiver, in a schematic view.

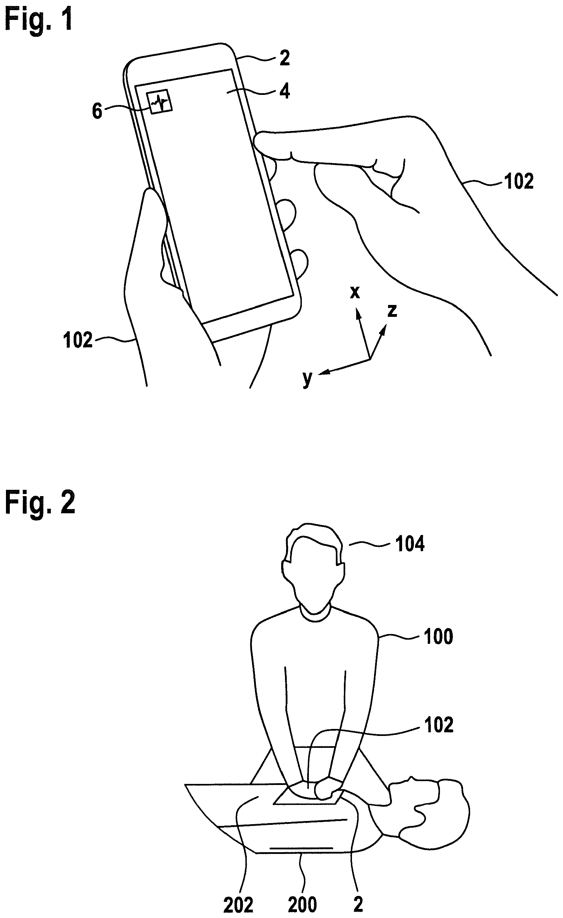

Fig. 1 shows a smartphone 2 in accordance with an exemplary embodiment of the invention in a perspective view. The smartphone 2 is depicted as being held and operated by the hands 102 of a user. In particular, the smartphone 2 is positioned in the user's left hand and operated by the user with his/her right hand.

The smartphone 2 has a display 4. The display 4 is embodied as a touchscreen, providing a visual output to the user and allowing the user to provide input to the smartphone 2. The display 4 covers a large portion of the front side of the smartphone 2. The display 4 may have a larger or smaller extension than shown in Fig. 1.

On the display 4, there is depicted a smartphone app icon 6. This smartphone app icon 6 links to a computer program in accordance with an exemplary embodiment of the invention. By touching the smartphone app icon 6 on the display 4, the user may start said computer program, which will then carry out a method for evaluating the effectiveness of CPR in accordance with an exemplary embodiment of the invention.

Fig. 1 further illustrates a coordinate system in the frame of reference of the smartphone 2. Said coordinate system is indicated with three arrows, illustrating the x-direction, the y-direction, and the z-direction in the smartphone frame of reference. The x-direction and the y-direction span a main plane of extension of the smartphone 2, with the main plane of extension coinciding / being parallel with the output surface of the display 4. The z-direction is orthogonal to the display 4 and faces towards the user of the smartphone 2. In other words, the positive z-direction extends out of the front side of the smartphone 2.

Fig. 2 shows the smartphone 2 of Fig. 1 placed on a patient 200. In particular, Fig. 2 shows the smartphone 2 placed on the chest 202 of the patient 200. In the illustration of Fig. 2, the patient 200 is in cardiac arrest and is in need of CPR. Before the start of the performance of CPR, the smartphone 2 is placed on that portion of the chest 202 of the patient 200 where compressive forces are applied. The smartphone 2 is placed on the chest 202 with the display 4 facing away from the chest 202. In other words, the positive z-direction in the smartphone frame of reference is pointing upwards in the earth frame of reference, assuming that the patient 200 is lying more or less flat on his back.

Fig. 2 further depicts a person 100 performing CPR on the patient 200. This person may also be referred to as caregiver 100. For performing CPR, the caregiver 100 kneels on the side of the patient 200 and places his/her hands 102 on the smartphone 2. The caregiver's head 104 faces the patient 200 and, thus, sees the smartphone 2 and the hands 102. The caregiver 100 may be the same person, whose hands 102 are depicted in Fig. 1 for operating the smartphone 2. Fig. 2 may depict the situation after the caregiver 100 has started the method for evaluating the effectiveness of CPR in accordance with an exemplary embodiment of the invention via touching the smartphone app icon 6, depicted in Fig. 1, after placing the smartphone 2 on the chest 202 of the patient 200, and after placing his/ her hands 102 on the smartphone 2. The caregiver 100 may now carry out CPR by repeatedly pressing the smartphone 2 into the chest 202 of the patient 200. During the CPR, the smartphone 2 may guide the caregiver 100 through the performance of CPR via at least one of visual outputs, acoustic outputs, and vibratory outputs. While the caregiver's hands 102 may cover a large portion of the front side of the smartphone 2, some portions of the display 4 of the smartphone 2 may be uncovered and may be available for providing visual outputs to the caregiver 100.

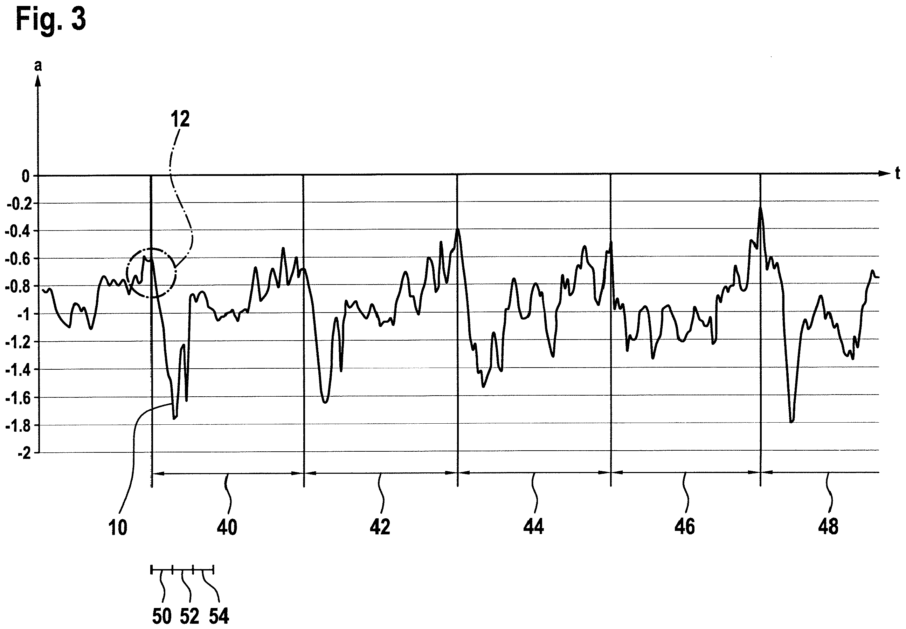

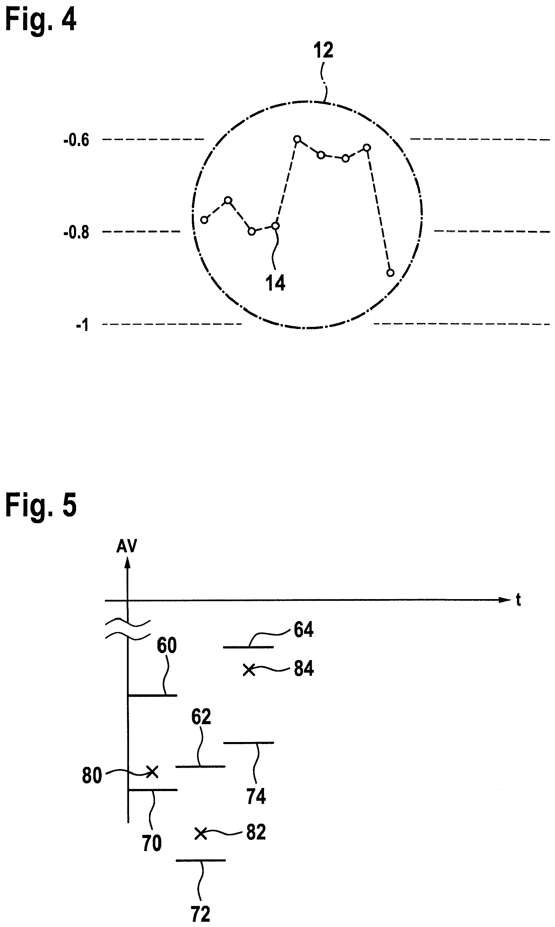

Fig. 3 depicts a sequence 10 of acceleration values, as obtained from acceleration data gathered by an acceleration sensor of a smartphone during CPR, when performed with the smartphone on the patient's chest, as for example illustrated in Fig. 2. The sequence 10 of acceleration values forms the basis for a method for evaluating the effectiveness of CPR in the smartphone in accordance with an exemplary embodiment of the invention. Such a method in accordance with an exemplary embodiment of the invention will be described below with reference to Fig. 3 and additional reference to Fig. 4, Fig. 5, and Fig. 6.

Fig. 3 depicts a sequence 10 of acceleration values over time. While the sequence 10 is depicted as a continuous curve in Fig. 3 for ease of illustration, it is understood that the sequence 10 of acceleration values is a sequence of discretized acceleration values. Subsequent acceleration values are provided at subsequent points in time, with a set time difference therebetween. This is illustrated with respect to Fig. 4, where individual acceleration values 14 are depicted as small circles and are connected to each other via a dashed line. Fig. 4 depicts the portion 12 of the sequence 10 of acceleration values of Fig. 3 in an enlarged view. The portion 12 is indicated via a circle in Fig. 3 and Fig. 4.

The sequence 10 of acceleration values are depicted as normalized with respect to the acceleration of gravity g, i.e. the sensed acceleration values are divided by 9.81 m/s² and the resulting acceleration values are depicted. Further, the sequence 10 of acceleration values are acceleration values in the z-direction of the smartphone. With the smartphone being placed on the patient's chest during CPR and the display pointing upwards, the acceleration of gravity g, which points towards the center of the earth, results in a measured acceleration of -1 on the scale of Fig. 3 in the absence of any compressive force by caregiver. This means that values above -1 are the result of an upward acceleration with respect to the patient and that values below -1 are the result of a downward acceleration with respect to the patient. It is pointed out that the sequence 10 of acceleration values may be represented in any other suitable manner. For example, the acceleration of gravity may be completely removed from the acceleration data when obtaining the sequence of acceleration values, i.e. 9.81 m/s² may be added to the sensed acceleration values, before the resulting sequence of acceleration values is provided. Other pre-processing steps may be performed as well, before the sequence of acceleration values is subject to the signal processing as described hereinbelow.

In Fig. 3, various CPR compression cycles are depicted. In particular, a first CPR compression cycle 40, a second CPR compression cycle 42, a third CPR compression cycle 44, a fourth CPR compression cycle 46, and a fifth CPR compression cycle 48 are indicated. It can be seen that the CPR compression cycles are similar in duration. Assuming that the ideal CPR frequency is 100 CPR compression cycles per minute according to medical guidelines, a single CPR compression cycle may be about 0.6 seconds in duration.

In the exemplary embodiment of Fig. 3, the beginning of a CPR compression cycle is defined as a sharp decline of the acceleration values. For example, such a decline may be defined as a decline of at least 0.2, before any increase in acceleration is observed, or may be defined as a decline of at least 1 over a certain time period, such as over a quarter of a CPR compression cycle, or may be defined in any other suitable manner. In the exemplary embodiment of Fig. 3, the beginning of a CPR compression cycle is used as the trigger for evaluating the effectiveness of CPR for a given CPR compression cycle. It is pointed out that other triggers are possible as well. It is further possible that an additional time condition is provided for determining the next trigger in the sequence 10 of acceleration values. For example, above described conditions of a sharp decline in acceleration may be combined with a condition of such sharp decline being present in a certain time window of the sequence 10 of acceleration values.

A method for evaluating the effectiveness of CPR in accordance with an exemplary embodiment of the invention will now be described with respect to the first CPR compression cycle 40, as indicated in Fig. 3. Starting from the beginning of the first CPR compression cycle 40, a subset of the sequence 10 of acceleration values are grouped into a first acceleration value block 50, a second acceleration value block 52, and a third acceleration value block 54. The lengths / durations of the first, second, and third acceleration value blocks 50, 52, 54 are indicated at the bottom of Fig. 3. In the exemplary embodiment of Fig. 3, each of the first, second, and third acceleration value blocks 50, 52, 54 contains the same number of acceleration values. In particular, each of the first, second and third acceleration value blocks 50, 52, 54 may have between 5 and 20 acceleration values. Jointly, the duration of the first, second, and third acceleration value blocks corresponds roughly to half the duration of the first CPR compression cycle 40. It may therefore be said that the first, second, and third acceleration value blocks 50, 52, 54 cover a pressure phase of a CPR compression cycle.

For each of the first, second, and third acceleration value blocks, a block-specific aggregate value is determined. In the exemplary embodiment of Figs. 3 to 6, the acceleration values of a given acceleration value block are summed, in order to yield the block-specific aggregate value. In the described exemplary embodiment, the individual acceleration values of a given acceleration value block are summed in an un-weighted manner. It is also possible to sum the individual acceleration values in a weighted manner. For example, early acceleration values in a given acceleration value block may be given larger weight than later acceleration values. It is also possible that a more complex aggregation function is applied to the acceleration values of a given acceleration value block. Irrespective of what kind of aggregation function is used, the multiple individual acceleration values of a given acceleration value blocks are transformed into a single block-specific aggregate value in the exemplary embodiment of Figs. 3 to 6.

For the first acceleration value block 50, a first block-specific aggregate value 80 is determined. For the second acceleration value block 52, a second block-specific aggregate value 82 is determined. For the third acceleration value block 54, a third block-specific aggregate value 84 is determined. The first, second, and third block-specific aggregate values 80, 82, 84 are depicted in Fig. 5 as subsequent aggregate values along a time axis. In the exemplary method illustrated in Figs. 3 to 6, each of the block-specific aggregate values 80, 82, 84 is compared with a respective upper block-specific threshold value and a respective lower block-specific threshold value. In particular, the first block-specific aggregate value 80 is compared with a first upper block-specific threshold value 60 and a first lower block-specific threshold value 70, the second block-specific aggregate value 82 is compared with a second upper block-specific threshold value 62 and a second lower block-specific threshold value 72, and the third block-specific aggregate value 84 is compared with a third upper block-specific threshold value 64 and a third lower block-specific threshold value 74. The upper block-specific threshold values 60, 62, 64 and the lower block-specific threshold values 70, 72, 74 are also illustrated in Fig. 5.

For the first CPR compression cycle 40, as indicated in Fig. 3, the first block-specific aggregate value 80 is between the first upper block-specific threshold value 60 and the first lower block-specific threshold value 70, the second block-specific aggregate value 82 is between the second upper block-specific threshold value 62 and the second lower block-specific threshold value 72, and the third block-specific aggregate value 84 is between the third upper block-specific threshold value 64 and the third lower block-specific threshold value 74. With the method determining that the block-specific aggregate values 80, 82, 84 are all between the respective upper block-specific threshold values and lower block-specific threshold values, the method concludes that an effective compression depth has been reached in the pressure phase of the first CPR compression cycle 40. In other words, the method determines an indication that an effective compression depth has been reached. Said indication may be output to the caregiver 100, illustrated in Fig. 2, by a green illumination of the display of the smartphone 2 or by a suitable speech output, such as "good depth", or in any other suitable manner.

The method, as described with respect to the first CPR compression cycle 40, may be repeatedly carried out, in particular carried out for each of the first CPR compression cycle 40, the second CPR compression cycle 42, the third CPR compression cycle 44, the fourth CPR compression cycle 46, the fifth CPR compression cycle 48, etc. In this way, the performance of CPR may be continuously evaluated, and a continuous feedback and guiding may be provided to the caregiver 100. It is also possible that similar method steps may be carried out for the release phase of the CPR compression cycles, with the release phases roughly corresponding to the respective second halves of the CPR compression cycles.

The upper block-specific threshold values 60, 62, 64 and the lower block-specific threshold value 70, 72, 74 are an exemplary pattern of threshold values, to which the sequence of acceleration values are related. It is pointed out that it is possible to relate aggregate values to the pattern of threshold values, as described above, and that it is possible to relate individual acceleration values to respective threshold values. The pattern of threshold values may extend along a full length of a CPR compression cycle or may extend along a portion thereof.

The pattern of threshold values may be set threshold values. It is also possible that the pattern of threshold values are threshold values that are dependent on basic patient information. For example, the method may receive basic patient data, such as sex and/or age and/or weight and/or an indication whether the patient is a child or an adult or an elderly person, and may select the pattern of threshold values in accordance with this basic patient data. The basic patient data may be input to the smartphone via the touchscreen, before the caregiver 100 starts performing CPR.

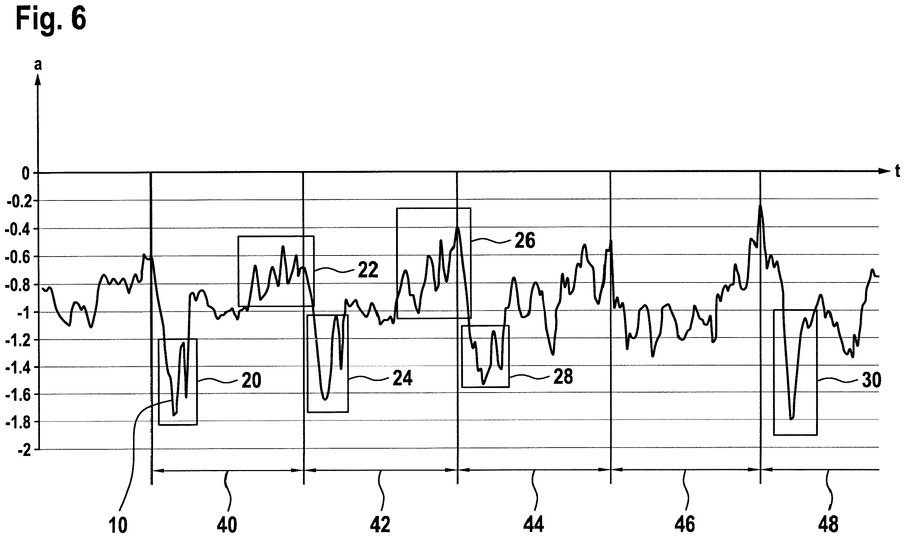

Fig. 6 shows the sequence 10 of acceleration values of Fig. 3 and illustrates an additional procedure for verifying that an effective compression step has been reached / for assessing the reliability of the indication whether an effective compression step has been reached, as determined by above described methods. This additional procedure involves an examining of the sequence 10 of acceleration values for predetermined acceleration spike patterns. On the basis of whether such acceleration spike patterns are found or not, the effected compression step may be verified.

In the pressure phase of the first CPR compression cycle 40, the sequence 10 of acceleration values has a first spike pattern 20. The method checks whether the first spike pattern 20 fulfils the conditions of a first predetermined acceleration spike pattern. The first predetermined acceleration spike pattern is defined as a spike pattern that takes place in a pressure phase of a CPR compression cycle, that has an initial acceleration spike, whose minimum is below -1.6 on the given scale, as described above, and that has a follow-up acceleration spike, whose minimum is below -1.4 on the given scale. For the first predetermined acceleration spike pattern, the initial acceleration spike and the follow-up acceleration spike are distinguished as two spikes, if an acceleration value above -1.4 on the given scale is present therebetween. The first acceleration spike pattern 20 has an initial acceleration spike, whose minimum is about -1.75, a second acceleration spike, whose minimum is about -1.62, and a maximum therebetween of about 1.22. Accordingly, the first spike pattern 20 fulfils all the criteria of the first predetermined acceleration spike pattern. As a consequence, the method determines that the indication that an effective compression depth has been reached, as discussed above, is reliable. Without wanting to be bound by theory, the inventor has found that the first predetermined acceleration spike pattern is characteristic, when pressing a smartphone into the chest of a patient, with the acceleration spikes being characteristic for the response of the human ribs to the pressure exerted during CPR. By examining the sequence of acceleration values for the first predetermined acceleration spike pattern and finding a spike pattern that matches the criteria of the first predetermined acceleration spike pattern, the method may conclude that the patient's chest is compressed in a desired manner and that the motion of the smartphone is not attributable to other circumstances, such as a soft ground underneath the patient.

In the release phase of the first CPR compression cycle 40, there is a second spike pattern 22. The second spike pattern 22 is mostly in the release phase of the first CPR compression cycle 40 and also extends somewhat into the pressure phase of the second CPR compression cycle 42. As the distinction between the pressure phase and the release phase depends on the definition of the beginning of the respective CPR compression cycle, as discussed above, the second spike pattern 22 is fairly characterized as being part of the first CPR compression cycle 40.

For the second spike pattern 22, the method examines whether the second spike pattern 22 fulfils the criteria of a second predetermined acceleration spike pattern. In the given exemplary embodiment, the second predetermined acceleration spike pattern is defined to substantially take place in the release phase of a CPR compression cycle, to have four acceleration spikes, whose maximum acceleration values are above -0.8 on the given scale, as discussed above, and to have local minimums below -0.8 between the four acceleration spikes on the given scale. The second acceleration spike pattern 22 fulfils these conditions and, thus, fulfils the criteria of a second predetermined acceleration spike pattern. The method may interpret the presence of the second predetermined acceleration spike pattern in the release phase of the first CPR compression cycle 40 as an additional reliability indicator as to the effectiveness of the performed CPR.

In the second CPR compression phase 42, there is a third acceleration spike pattern 24 in the pressure phase and a fourth acceleration spike pattern 26 substantially in the release phase. Again, both the third acceleration spike pattern 24 and the fourth acceleration spike pattern 26 meet the criteria of the first predetermined acceleration spike pattern and the second predetermined acceleration spike pattern. Assuming that the method determined an indication that an effective compression depth has been reached for the second CPR compression cycle 42 on the basis of the block-specific aggregate values, the method further finds said indication to be reliable due to the presence of both the first and the second predetermined acceleration spike patterns. While the method is described to examine the sequence 10 of acceleration values for both the first and the second predetermined acceleration spike patterns, it is pointed out that the method may also examine the sequence 10 of acceleration values for only one of the first and the second predetermined acceleration spike patterns.

In the pressure phase of the third CPR compression cycle 44, there is a fifth acceleration spike pattern 28. The fifth acceleration spike pattern 28 does not fulfil the criteria of the first predetermined acceleration spike pattern, because the first spike does not have a minimum below -1.6 on the given scale. Also, the release phase of the third CPR compression cycle 44 does not have an acceleration spike pattern that somehow resembles the second predetermined acceleration spike pattern. Accordingly, even if the method had determined from the acceleration values of the third CPR compression cycle 44 that an effective compression depth has been reached, the method would determine from the lack of predetermined acceleration spike patterns that such result would not be reliable. The same is true for the fourth CPR compression cycle 46, where no acceleration spike pattern that is close to the first predetermined acceleration spike pattern or to the second predetermined acceleration spike pattern is present. In the absence of acceleration spikes fulfilling the criteria of the first predetermined acceleration spike pattern and/or the second predetermined acceleration spike pattern, the method may determine that no effective compression depth has been reached and may give the user feedback to push deeper into the patient's chest. It is also possible that the method considers such a CPR compression cycle without the first and/or second predetermined acceleration spike pattern to be inconclusive and to wait for the next CPR compression cycle.

In the pressure phase of the fifth CPR compression cycle 48, there is a sixth acceleration spike pattern 30. While the initial spike of this sixth acceleration spike pattern 30 fulfils the criterion of having a minimum below -1.6 on the given scale, the follow-up acceleration spike does not have a minimum below -1.4 on the given scale. As a result, the method may also deem an indication that an effective compression depth has been reached, should the same have been determined, to be not reliable.

While the predetermined acceleration spike patterns have been described as being a means of verification regarding the indication that an effective compression depth has been reached, the examining of the sequence of acceleration values for one or more predetermined acceleration spike pattern(s) may also form, by itself, the basis for determining an indication whether an effective compression depth has been reached. In other words, an indication whether an effective compression depth has been reached may also be determined on the basis of the examining of the sequence of acceleration values for one or more predetermined acceleration spike pattern(s), without relating the sequence of acceleration values to the pattern of threshold values.

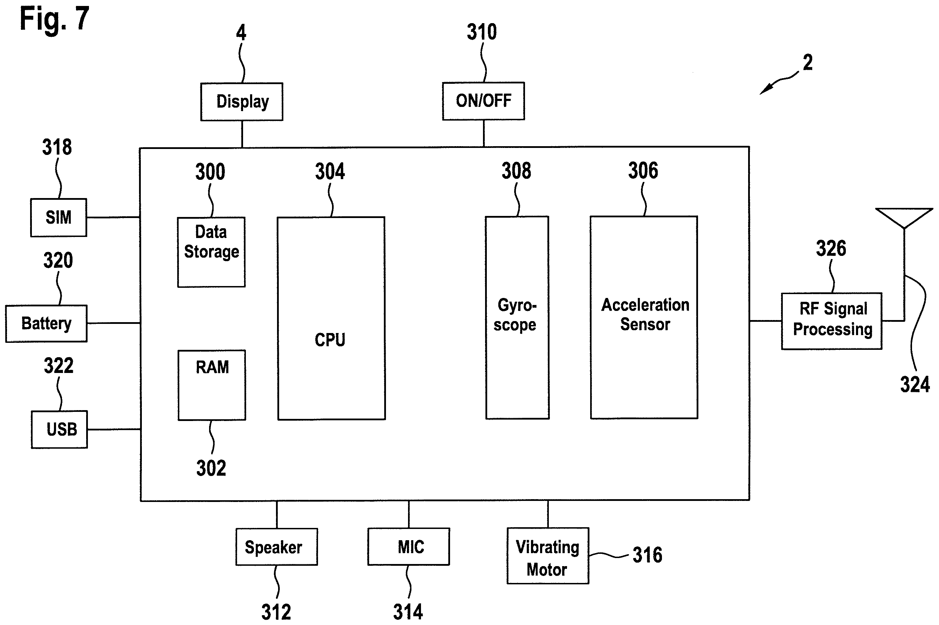

Fig. 7 shows a smartphone 2 in accordance with an exemplary embodiment of the invention in a block diagram. As internal components, the smartphone 2 comprises a data storage 300, a random access memory 302, a central processing unit 304, an acceleration sensor 306, and a gyroscope 308. A computer program in accordance with an exemplary embodiment of the invention may be stored in the data storage 300 and may be loaded into the random access memory 302 upon being started by a user. The program instructions of the computer program may be carried out by the CPU 304, with the program instructions causing the smartphone 2 to carry out a method for evaluating the effectiveness of CPR in accordance with an exemplary embodiment of the invention. As described above, the CPU 304 may read in acceleration data from the acceleration sensor 306 and gyroscope data from the gyroscope 308 during executing the computer program. It is understood that the smartphone 2 may have a different internal component structure and/or may have a variety of further internal components not shown in Fig. 7.

For interaction with the smartphone environment, the smartphone 2 has a display 4, an on/off switch 310, one or more speakers 312, a microphone 314, a vibrating motor 316, a SIM card slot 318, a battery 320, which may also be an internal component, a USB port 322, which may be provided for data communication with the outside world and/or for recharging the battery 320, an antenna 324, and a radio frequency signal processing unit 326. It is understood that the smartphone 2 may have a different component structure for interaction with the smartphone environment and/or may have various further components for interaction with the smartphone environment.

When executing the computer program in accordance with an exemplary embodiment of the invention, the CPU 304 may cause the display 4 and/or the speaker 312 and/or the vibrating motor 316 to provide a visual output and/or an acoustic output and/or a vibratory output to the user of the smartphone 2. Solely on the basis of the acceleration data gathered by the acceleration sensor 306 and provided to the CPU 304, potentially in combination with the gyroscope data from the gyroscope 308, the method in accordance with exemplary embodiments of the invention may guide a user of the smartphone 2 through the performance of CPR and may provide valuable feedback to the user 2 to carry out the CPR with an effective compression frequency and/or an effective compression depth during the individual CPR compression cycles.

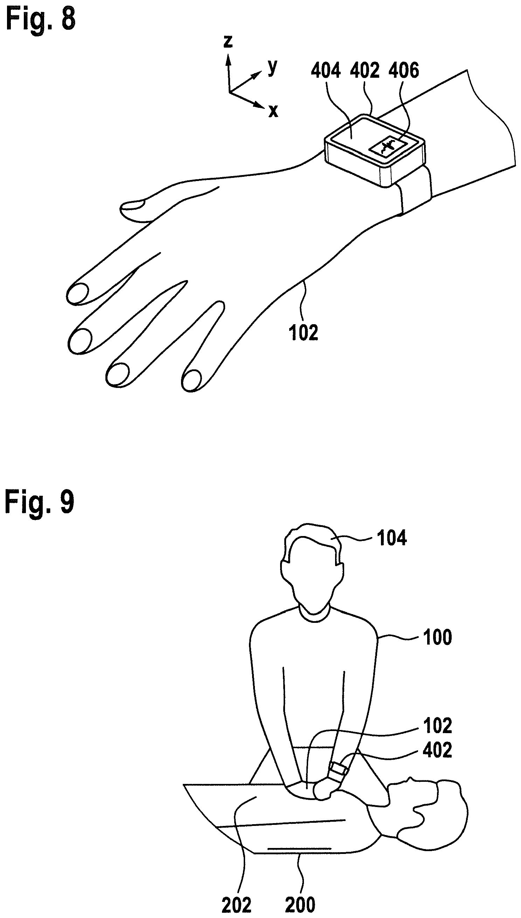

Fig. 8 shows a smartwatch 402 in accordance with an exemplary embodiment of the invention in a schematic perspective view. The smartwatch 402 is depicted as being worn by a user. In particular, the smartwatch 402 is positioned on a user's wrist, adjacent to his/her left hand 102.

The smartwatch 402 has a display 404. The display 404 is embodied as a touchscreen, providing a visual output to the user and allowing the user to provide input to the smartwatch 402. The display 404 covers a large portion of the front side of the smartwatch 402. The display 404 may have a larger or smaller extension than shown in Fig. 8.

On the display 404, there is depicted a smartwatch app icon 406. This smartwatch app icon 406 links to a computer program in accordance with an exemplary embodiment of the invention. By touching the smartwatch app icon 406 on the display 404, the user may start said computer program, which will then carry out a method for evaluating the effectiveness of CPR in accordance with an exemplary embodiment of the invention.

Fig. 8 further illustrates a coordinate system in the frame of reference of the smartwatch 402. Said coordinate system is indicated with three arrows, illustrating the x-direction, the y-direction, and the z-direction in the smartwatch frame of reference. The x-direction and the y-direction span a main plane of extension of the smartwatch 402, with the main plane of extension coinciding / being parallel with the output surface of the display 404. The z-direction is orthogonal to the display 404 and faces towards the user of the smartwatch 402. In other words, the positive z-direction extends out of the front side of the smartwatch 402.

The exemplary smartwatch 402 may have components identical or similar to the smartphone described herein. In particular, the components described with respect to Fig. 7 may be present in the exemplary smartwatch 402 as well. It is understood that this component set-up is exemplary only and that the smartwatch 402 may have a different set-up and/or additional components.

Fig. 9 shows the smartwatch 402 of Fig. 8, placed around the wrist of a caregiver 100, during performance of CPR on a patient 200 by the caregiver 100. The situation is similar to the situation depicted in Fig. 2. However, instead of the smartphone 2 being placed on the chest 202 of the patient 200 and the caregiver 100 pressing the smartphone 2 into the patient's chest 202, the caregiver 100 performs CPR on the patient 200 with his/her bare hands, while wearing the smartwatch 402 on his/her wrist. The smartwatch 402 may also be worn on the right hand. Fig. 9 may depict the situation after the caregiver 100 has started the method for evaluating the effectiveness of CPR in accordance with an exemplary embodiment of the invention via touching the smartwatch app icon 406, depicted in Fig. 8. The caregiver 100 may now carry out CPR by repeatedly pushing his/ her hands into the chest 202 of the patient 200. During the CPR, the smartwatch 402 may guide the caregiver 100 through the performance of CPR via at least one of visual outputs, acoustic outputs, and vibratory outputs.

When the caregiver 100 performs CPR, the smartwatch 402 moves in the same or a very similar manner as the caregiver's hands 102. Accordingly, the method for evaluating the effectiveness of CPR, as described above with respect to Figs. 3 to 6, making reference to the exemplary sequence 10 of acceleration values, may be carried out in the smartwatch 402 in an analogous or at least highly similar manner. Instead of using the acceleration values in the z-direction of the smartphone, acceleration values in an estimated compression direction may be used. The estimated compression direction may for example be the upwards / downwards direction in the earth frame of reference or may be the direction of largest acceleration values, as sensed by the acceleration sensor of the smartwatch 402.

While the invention has been described with reference to exemplary embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed, but that the invention will include all embodiments falling within the scope of the appended claims.

## Claims

1. Method for evaluating the effectiveness of CPR in a smartphone (2) or smartwatch (402), the method comprising:
obtaining a sequence (10) of acceleration values (14) from acceleration data gathered by an acceleration sensor (306) of the smartphone (2) or smartwatch (402);
relating the sequence (10) of acceleration values (14) to a pattern of threshold values (60, 62, 64, 70, 72, 74); and
on the basis of said relating of the sequence (10) of acceleration values (14) to the pattern of threshold values (60, 62, 64, 70, 72, 74), determining an indication whether an effective compression depth has been reached.

2. Method according to claim 1, wherein the pattern of threshold values (60, 62, 64, 70, 72, 74) comprises upper threshold values and lower threshold values.

3. Method according to claim 1 or 2, wherein said relating of the sequence (10) of acceleration values (14) to the pattern of threshold values (60, 62, 64, 70, 72, 74) comprises:
grouping a subset of the sequence of acceleration values into a plurality of acceleration value blocks (50, 52, 54);
for each of the plurality of acceleration value blocks (50, 52, 54), determining a block-specific aggregate value (80, 82, 84); and
for each of the block-specific aggregate values (80, 82, 84), comparing the block-specific aggregate value with at least one block-specific threshold value.

4. Method according to claim 3, wherein said determining of the block-specific aggregate value (80, 82, 84) comprises applying an aggregation function, to the acceleration values in the given acceleration value block (50, 52, 54), wherein said applying the aggregation function in particular comprises forming a sum of the acceleration values in the given acceleration value block (50, 52, 54).

5. Method according to claim 3 or 4, wherein said comparing of the block-specific aggregate value (80, 82, 84) with at least one block-specific threshold value comprises determining whether the block-specific aggregate value (80, 82, 84) is between a respective upper block-specific threshold value (60, 62, 64) and a respective lower block-specific threshold value (70, 72, 74), and wherein said determining of the indication whether an effective compression depth has been reached in particular comprises determining whether the block-specific aggregate values (80, 82, 84) of all acceleration value blocks (50, 52, 54) of a pressure phase of a CPR compression cycle are between the respective upper block-specific threshold values (60, 62, 64) and the respective lower block-specific threshold values (70, 72, 74).

6. Method according to any of the preceding claims, wherein a sampling rate of the sequence (10) of acceleration values (14) is at least 50 Hz, in particular between 50 Hz and 500 Hz, further in particular between 100 Hz and 200 Hz.

7. Method according to any of the preceding claims, wherein said obtaining of the sequence (10) of acceleration values (14) from acceleration data gathered by the acceleration sensor (306) of the smartphone (2) comprises obtaining a sequence of acceleration values in the z-direction of the smartphone (2).

8. Method according to claim 7, wherein obtaining the sequence (10) of acceleration values (14) in the z-direction of the smartphone (2) comprises using gyroscope data, gathered by a gyroscope (308) of the smartphone (2), for determining a tilt of the smartphone (2).

9. Method according to any of the preceding claims, further comprising:
examining the sequence (10) of acceleration values (14) for a predetermined acceleration spike pattern; and
on the basis of said examining of the sequence of acceleration values for the predetermined acceleration spike pattern, assessing a reliability of the indication whether an effective compression depth has been reached.

10. Method according to claim 9, wherein the predetermined acceleration spike pattern comprises an initial acceleration spike in a pressure phase of a CPR compression cycle and a follow-up acceleration spike in the same pressure phase.

11. Method according to any of the preceding claims, further comprising:
determining at least one of a CPR compression cycle duration and a CPR compression cycle frequency from the sequence (10) of acceleration values (14).

12. Method according to any of the preceding claims, further comprising:
providing at least one of an acoustic output, a visual output, and a vibratory output via the smartphone (2) or smartwatch (402), the at least one of the acoustic output, the visual output, and the vibratory output being based on at least one of the indication whether an effective compression depth has been reached, the CPR compression cycle duration, and the CPR compression cycle frequency.

13. Method of performing CPR on a patient (200) in cardiac arrest, comprising:
placing a smartphone (2) on the chest (202) of the patient (200);
pressing the smartphone (2) into the chest (202) of the patient (200);
evaluating the effectiveness of the CPR in the smartphone (2) in accordance with the method of any of the preceding claims; and
providing feedback regarding the effectiveness of the CPR via at least one of an acoustic output, a visual output, and a vibratory output from the smartphone.

14. Computer program for a smartphone (2) or smartwatch (402), the computer program containing program instructions which, when executed on a smartphone (2) or smartwatch (402), carry out a method for evaluating the effectiveness of CPR in accordance with any of claims 1-12.

15. Smartphone (2) or smartwatch (402) configured to perform a method for evaluating the effectiveness of CPR in accordance with any of claims 1-12.
